# FASCICULE DE BREVET EUROPEEN

(11) **EP 2 908 763 B1**
(45) Date de publication et mention de la délivrance du brevet: **04.01.2017**
(21) Numéro de dépôt: 13782791.1
(22) Date de dépôt: 03.10.2013
(51) Int. Cl.: A61C 17/20, A61C 17/02, A61B 17/00, A61C 1/07, G05B 15/02, A61C 1/00, A61B 17/16

(54) **DISPOSITIF DE CONTRÔLE D'UNE PIECE A MAIN CHIRURGICALE**
VORRICHTUNG ZUR STEUERUNG EINES CHIRURGISCHEN HANDSTÜCKS
DEVICE FOR CONTROLLING A SURGICAL HANDPIECE

(30) Priorité: 18.10.2012 FR 1259927
(43) Date de publication de la demande: 26.08.2015
(73) Titulaire: SOCIETE POUR LA CONCEPTION DES APPLICATIONS DES TECHNIQUES ELECTRONIQUES, 33700 Merignac (FR)
(72) Inventeur: REGERE, Pascal, 33290 Blanquefort (FR); RECHE, Charles, 33000 Bordeaux (FR); GALLARD, Yann, 33185 Le Haillan (FR)
(74) Mandataire: Desormiere, Pierre-Louis
(86) Numéro de dépôt international: PCT/FR2013/052355
(87) Numéro de publication internationale: WO 2014/060676

(56) Documents cités:
- WO-A1-2011/141442
- US-A1- 2007 254 261

## Description

### Arrière-plan de l'invention

La présente invention concerne le domaine des pièces à main chirurgicales telles que celles utilisées dans les soins dentaires ou médicaux, et concerne plus particulièrement un appareil de contrôle d'une telle pièce à main.

La présente invention s'applique notamment aux détartreurs, micromoteurs, appareils de prophylaxie, de diagnostic, d'imagerie ou de chirurgie.

L'encombrement des cabinets dentaires ou médicaux par le matériel utilisé par le praticien est un problème récurrent dû à la fois aux nombreuses liaisons filaires des cordons d'alimentation des pièces à mains chirurgicales en électricité ou en fluides et aux multiples liaisons des systèmes de commandes associés.

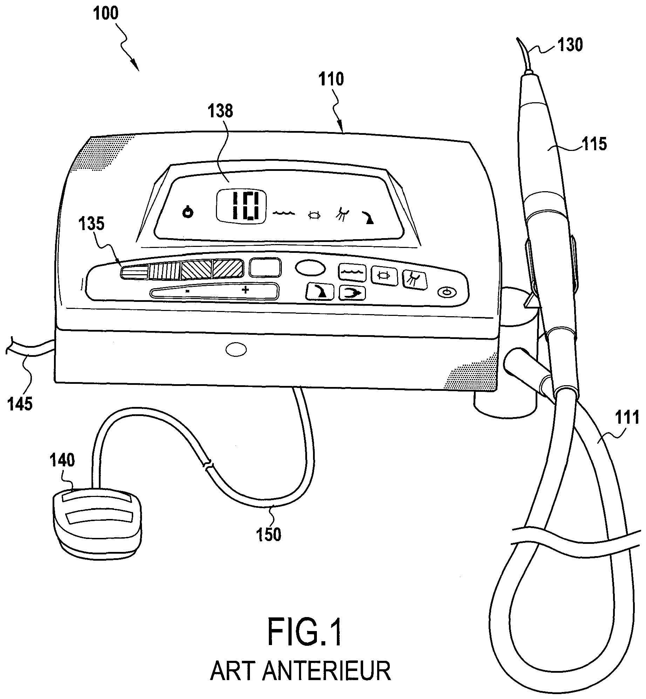

C'est le cas par exemple en chirurgie-dentaire pour les appareils de détartrage. Comme représenté en **figure 1****,** un appareil de détartrage 100 se compose typiquement d'un dispositif de contrôle 110 (i.e. l'unité centrale) servant notamment de générateur d'ultrasons, celui-ci étant raccordé à la pièce à main chirurgicale 115 par un cordon 111. Une sonotrode ou insert ultrasonique 130 est monté sur la partie supérieure ou distale de la pièce à main 115. De façon bien connue, la pièce à main 115 comprend un transducteur piézoélectrique (non représenté) couplé mécaniquement à l'insert 130 de manière à transmettre à ce dernier des vibrations ultrasonores dont les amplitudes sont fonction de la puissance électrique fournie par le générateur d'ultrasons.

Le dispositif de contrôle 110 peut en outre alimenter la pièce à main 115 en fluide d'irrigation à travers le cordon 111 à l'aide d'une pompe d'irrigation (non représentée).

La face avant du dispositif de contrôle 110 comporte ici une interface homme-machine constituée de touches 135 et d'un écran 138. Cette interface permet à l'opérateur de régler les paramètres de contrôle tels que la puissance électrique fournie à la pièce à main 115 et le débit d'irrigation généré par la pompe d'irrigation.

Le système est en outre fréquemment équipé d'une pédale de commande filaire 140 permettant à l'opérateur de régler les paramètres de contrôle avec le pied.

Un tel appareil de détartrage comporte donc trois types de raccordements, à savoir : un première raccordement pour l'alimentation du dispositif de contrôle 110, un deuxième raccordement (i.e. le cordon 111) pour relier la pièce à main 115 au dispositif de contrôle 110 et un troisième raccordement 150 reliant le dispositif de contrôle 110 à la pédale 140.

L'augmentation du nombre des équipements présents dans les cabinets dentaires ou médicaux a rendu nécessaire la rationalisation de leur connectique et la réduction de leur encombrement.

Des solutions ont été apportées, en particulier en utilisant des dispositifs de contrôle capables de communiquer par une liaison sans fil de type monodirectionnelle ou bidirectionnelle avec une commande distante. Ce type de liaison sans fil peut fonctionner selon divers standards, tel qu'un protocole bluetooth™ (marque déposée) par exemple (conforme à la norme IEEE 802.15.1).

D'autres protocoles peuvent toutefois être utilisés tels que les protocoles Wifi, Zigbee et NFC correspondant respectivement aux normes IEEE 802.11, IEEE 802.15.4 et ISO/CEI 1444).

La commande à distance permet avantageusement de supprimer certaines liaisons filaires telles que celle qui relie la pédale de commande à l'unité centrale par exemple. Ce cas particulier est par exemple envisagé dans le document US 2012/0064483 A1.

WO2011/141442 A1 décrit une interface sans fil entre un contrôleur d'instruments chirurgicaux et un ou plusieurs smartphones.

Toutefois, les dispositifs de contrôle sans fil de pièce à main chirurgicale présentent certains risques en matière de sécurité liés à l'utilisation même de ces appareils.

En effet, il peut arriver que le dispositif de contrôle rencontre un disfonctionnement et que sa liaison de communication sans fil se dégrade, se coupe ou ne puisse s'établir correctement avec la commande distante. Une telle défaillance peut résulter de divers problèmes tels qu'un bug logiciel, un défaut de batterie, une erreur de l'opérateur... Il peut également arriver que la liaison sans fil soit perturbée par l'environnement extérieur (parasitage résultant de dispositifs sans fil environnants par exemple).

Il peut aussi arriver qu'il y ait un conflit entre deux systèmes de commande en parallèle filaire et non filaire.

D'autres situations d'urgence peuvent également survenir si la commande distante n'est pas à portée de l'opérateur (ou si cette commande distante est actionnée par erreur par un tiers) et que ce dernier souhaite rapidement reprendre le contrôle de la pièce à main.

Les interventions médicales réalisées avec ces pièces à main chirurgicales sont en général délicates de sorte qu'il est impératif que l'opérateur ait une parfaite maîtrise des paramètres de contrôle de l'appareil.

Il existe donc un besoin pour un dispositif de contrôle palliant les inconvénients des dispositifs actuels et, en particulier, pour un dispositif de contrôle facilitant le réglage des paramètres de contrôle et offrant un contrôle plus sécurisé de la pièce à main.

### Objet et résumé de l'invention

A cet effet, la présente invention concerne un dispositif de contrôle d'une pièce à main chirurgicale, apte à contrôler la pièce à main chirurgicale selon au moins un paramètre de contrôle, le dispositif de contrôle comprenant :
- des moyens de commande locaux présents sur le dispositif de contrôle ;
- des moyens de communication sans fil aptes à communiquer avec des moyens de commande distants par une liaison de communication sans fil, les moyens de commande locaux et les moyens de communication sans fil étant aptes à recevoir des commandes de réglage de chaque paramètre de contrôle ; et
- des moyens de sélection pour sélectionner soit les moyens de commande locaux soit les moyens de communication sans fil pour le réglage de chaque paramètre de contrôle.

L'invention permet avantageusement de sécuriser le réglage des paramètres de contrôle de la pièce à main chirurgicale. Grâce à l'invention, il est en particulier toujours possible pour l'opérateur de reprendre rapidement le contrôle de la pièce à main lorsqu'une défaillance survient au niveau de la communication sans fil ou lors d'une quelconque situation d'urgence (comme expliqué précédemment, les situations d'urgence peuvent être multiples).

Le dispositif de contrôle de l'invention permet avantageusement de définir des niveaux de priorité entre les moyens de commande locaux et les moyens de commande distants : en cas de défaillance par exemple dans la réception ou le traitement des commandes de réglages émises par les moyens de commande distants, il est toujours possible pour l'opérateur de reprendre la main sur le contrôle de la pièce à main.

Les moyens de sélection permettent en effet de définir quels moyens parmi les moyens de commande locaux et les moyens de communication sans fil doivent être pris en compte par le dispositif de contrôle de l'invention pour le réglage des paramètres de contrôle de la pièce à main. Lorsque notamment les moyens de commande locaux sont sélectionnés, les commandes reçues éventuellement par les moyens de communication sans fil de l'invention sont ignorés ce qui permet d'éviter tout réglage erroné de la pièce à main. Le contrôle de la pièce à main est ainsi plus sécurisé.

Selon un premier mode de réalisation, les moyens de commande locaux comprennent une partie amovible,
le dispositif de contrôle comprenant en outre des moyens de détection de la présence de la partie amovible sur le dispositif de contrôle,
les moyens de sélection étant configurés pour sélectionner automatiquement les moyens de commande locaux pour le réglage de chaque paramètre de contrôle en cas de détection de la présence de ladite partie amovible.

Dans ce premier mode de réalisation, dès que la partie amovible est en position de travail, les moyens de commande locaux ont la priorité sur les moyens de commande distants, i.e. les moyens de commandes locaux sont sélectionnés par les moyens de sélection. En conséquence, les commandes émises par le terminal sont ignorées, seules les commandes provenant des moyens de commande locaux étant alors prises en compte pour le réglage de la pièce à main.

Selon un deuxième mode de réalisation, les moyens de sélection comprennent un commutateur activable manuellement pour sélectionner soit les moyens de commande locaux soit les moyens de communication sans fil pour le réglage de chaque paramètre de contrôle.

Dans ce deuxième mode de réalisation, l'opérateur peut à tout moment commander, à l'aide du commutateur, la sélection soit des moyens de commande locaux, soit des moyens de communication à distance. Ainsi, en cas de défaillance de la liaison de communication sans fil (ou de tout autre situation d'urgence), l'opérateur peut rapidement reprendre la main sur la pièce à main chirurgicale en sélectionnant les moyens de commande locaux pour le réglage des paramètres de contrôle. De cette manière, le dispositif de contrôle est forcé d'ignorer toutes commandes pouvant provenir des moyens de commande distants via la liaison de communication sans fil.

Par ailleurs, la liaison de communication sans fil peut être de type bluetooth™ ou selon l'un des protocoles alternatifs déjà énumérés ci-avant (Wifi, Zigbee et NFC).

De plus, les moyens de commande locaux peuvent comprendre un bouton magnétique à effet hall.

Dans un mode de réalisation particulier, la pièce à main comprend un transducteur piézoélectrique, ledit au moins un paramètre comprenant au moins l'un parmi une puissance électrique fournie à la pièce à main, un débit d'irrigation de la pièce à main et l'activation ou désactivation de la pièce à main.

Corrélativement, l'invention concerne un appareil de contrôle d'une pièce à main chirurgicale comprenant :
- un dispositif de contrôle tel que défini ci-avant ;
- une pièce à main dont les paramètres de contrôle sont réglés par le dispositif de contrôle ; et
- des moyens de commande distants aptes à communiquer avec les moyens de communication sans fil du dispositif de contrôle par une liaison sans fil.

Les moyens de commande distants peuvent être un module de communication de type tablette numérique, téléphone mobile ou tous autres dispositifs équivalents.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures:
- la figure 1 déjà décrite ci-dessus représente, de manière schématique, un exemple d'appareil de soin dentaire comportant une pièce à main chirurgicale ;
- les figure 2, 3 et 4 représentent, de manière schématique, un dispositif de contrôle d'une pièce à main chirurgicale selon un premier mode de réalisation de l'invention ;
- la figure 5 représente, de manière schématique, une variante du premier mode de réalisation représenté en figures 3 et 4 ; et
- les figures 6 et 7 représentent, de manière schématique, un dispositif de contrôle d'une pièce à main chirurgicale selon un deuxième mode de réalisation de l'invention.

### Description détaillée de plusieurs modes de réalisation

Comme indiqué précédemment, l'invention concerne le domaine des pièces à main chirurgicales telles que celles utilisées dans les soins dentaires ou médicaux et concerne plus particulièrement un dispositif de contrôle d'une telle pièce à main.

Dans la suite de ce document, les exemples de mise en oeuvre de l'invention portent sur le cas particulier d'un dispositif de contrôle d'une pièce à main chirurgicale adaptée pour le détartrage. On comprendra toutefois que l'invention s'applique plus généralement à tous dispositifs de contrôle d'une pièce à main chirurgicale.

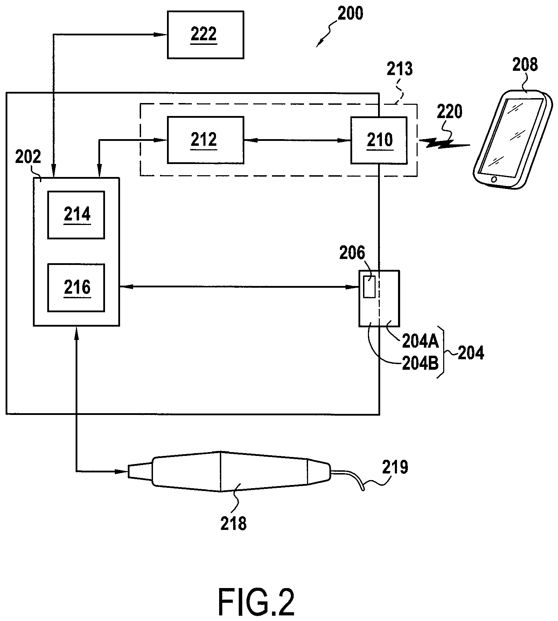

Un premier mode de réalisation de l'invention est à présent décrit en référence au dispositif de contrôle 200 représenté aux **figures 2****,** **3 et 4****.**

Le dispositif de contrôle 200 envisagé ici assure le contrôle d'une pièce à main chirurgicale 218 adaptée aux soins dentaires, tel que le détartrage par exemple. Cette pièce à main chirurgicale 218 comprend notamment un transducteur piézoélectrique mécaniquement contraint (non représenté) apte à générer et transmettre des vibrations ultrasonores à un insert 219 monté à l'extrémité distale de la pièce à main.

Le dispositif de contrôle 200 comprend une carte de gestion 202 correspondant dans cet exemple à une carte mère munie d'un processeur et de mémoires. L'architecture matérielle de cette carte est conventionnelle et ne sera donc pas décrite davantage dans ce document.

La carte mère 202 assure le fonctionnement général du dispositif de contrôle 200 et comprend notamment un module de contrôle 214 en charge du réglage des paramètres de contrôle de la pièce à main chirurgicale 218. Dans le cas présent, les paramètres contrôlés par la carte mère 202 peuvent comprendre au moins l'un parmi : la puissance électrique fournie à la pièce à main et le débit d'irrigation en fluides vers la pièce à main 218. En réglant le débit d'irrigation de manière adéquate (mise en route rapide et continue de la pompe), il est notamment possible de déclencher la purge de la pièce à main.

Le dispositif de contrôle 200 comprend également une carte fille de gestion 212 couplée à la carte mère. Cette carte fille 212 coopère avec l'interface de communication sans fil 210 et permet l'établissement d'une liaison de communication sans fil entre l'interface 210 et des entités externes au dispositif de contrôle 200. On envisagera dans cet exemple le cas où une liaison de communication bluetooth™ est établie entre l'interface de communication sans fil 210 et un terminal distant 208.

On comprendra néanmoins que des protocoles de communication sans fil autres que le bluetooth™ peuvent être envisagés dans le cadre de l'invention (tels que les protocoles Wifi, Zigbee et NFC comme déjà mentionnés précédemment). La liaison de communication 220 est de préférence de type radiofréquence mais d'autres technologies de communication telles que l'infrarouge sont également possibles.

La carte fille 212 est apte à recevoir et traiter des commandes de réglage de chaque paramètre de contrôle de la pièce main 218, ces commandes étant émises depuis le terminal distant 208 via la liaison de communication sans fil 220 puis transmises par l'interface de communication 210 à la carte fille 212. La carte fille 212 fait ici notamment office de décodeur de la liaison sans fil.

Le terminal 208 constitue ici des moyens de commande distants au sens de l'invention. Ce terminal 208 peut prendre la forme d'un module de communication, par exemple de type téléphone mobile ou tablette numérique. Une application logicielle exécutée par le téléphone permet à un opérateur de commander à distance les paramètres de contrôle de la pièce à main 218.

Alternativement, les moyens de commande distants 208 peuvent prendre la forme de tout autre type de commande sans fil, telle que par exemple celle d'une pédale de commande classique apte à émettre des commandes de réglage via une liaison de communication sans fil.

La carte fille 212 et l'interface de communication 210 constituent par ailleurs des moyens de communication sans fil 213 au sens de l'invention aptes à communiquer avec les moyens de commande distants (i.e. le terminal 208 dans cet exemple) par la liaison de communication sans fil 220.

La carte fille 212 transmet ensuite les commandes reçues à la carte mère 202 après, le cas échéant, avoir réalisé les prétraitements nécessaires.

On notera que, dans le mode de réalisation considéré ici, la carte fille 212 est distincte de la carte mère 202. Cette configuration n'est toutefois pas obligatoire et ne constitue qu'un mode de réalisation possible de l'invention. Alternativement, la fonction de la carte fille 212 peut être exécutée par la carte mère 202 elle-même.

Le dispositif de contrôle 200 comprend en outre des moyens de commande locaux 204 présents sur le dispositif de contrôle 200 lui-même. Le praticien est capable, à partir de ces moyens de commande locaux, de régler un ou plusieurs des paramètres de contrôle de la pièce à main 218.

Dans ce premier mode de réalisation, les moyens de commande locaux 204 comportent une partie amovible 204A et une partie fixe 204B d'un bouton magnétique à effet hall. La partie fixe 204B est solidaire du dispositif de contrôle 200 et peut coopérer avec la partie amovible 204A lorsque cette dernière est montée sur la partie fixe 204B. Dans cet exemple, le praticien peut enlever la partie amovible 204A manuellement puis la remonter sur la partie fixe 204B lorsqu'il souhaite régler les paramètres de contrôle de la pièce à main 218 directement sur le boîtier du dispositif de contrôle 200.

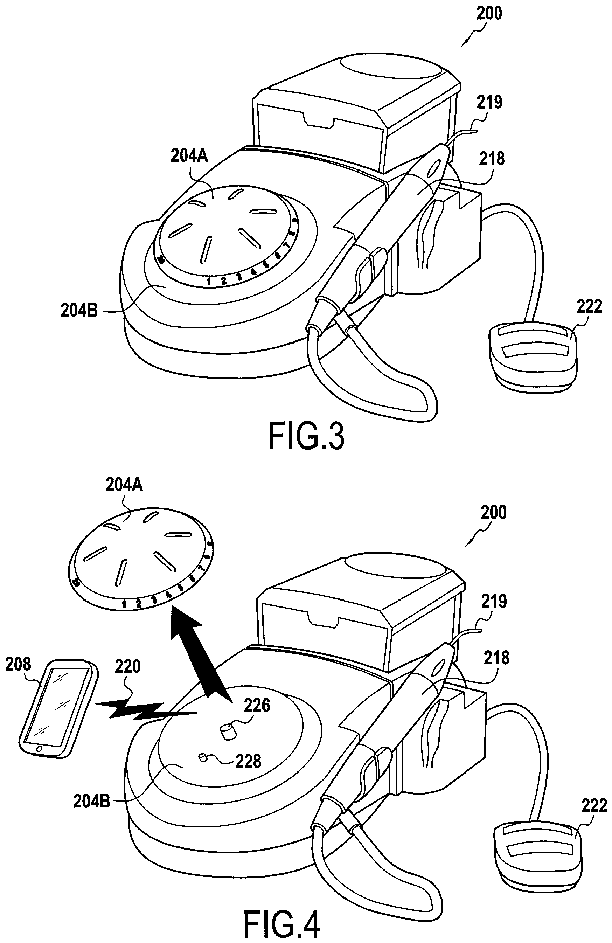

Comme représenté en **figure 4****,** la partie fixe 204B est ici équipée d'un ergot 226 configuré pour s'engager dans un renfoncement de la partie arrière de la partie amovible 204A lorsque cette dernière est en position de travail sur le dispositif de contrôle 200. L'opérateur peut alors régler un ou plusieurs paramètres de contrôle par rotation de la partie amovible 204A en s'aidant par exemple des repères visuels pouvant être disposés sur la partie amovible 204A (ou éventuellement sur la partie fixe 204B).

La partie fixe 204B comprend en outre une butée 228 qui permet de bloquer en rotation la rotation de la partie amovible 204A lorsque celui-ci se trouve en bout de course (i.e. lorsque l'opérateur a atteint la valeur de réglage maximale ou minimale possible).

En détachant la partie amovible 204A, l'utilisateur peut nettoyer efficacement la face avant du dispositif de contrôle 200 notamment au niveau de la partie fixe 204B et ainsi maintenir une bonne hygiène au dispositif de contrôle 200, ce qui est souvent critique dans le domaine médical.

On comprendra toutefois que des variantes de ce premier mode de réalisation sont possibles où le bouton magnétique à effet hall est remplacé par tout autre type de bouton ou de commande locale comportant une partie fixe 204B et une partie amovible 204A. Les moyens de commande locaux 204 peuvent par exemple correspondre à un bouton électrique ou à un bouton fonctionnant à l'aide de moyens optiques. Un bouton de type optique peut, par exemple, comprendre une LED montée dans la partie amovible et un ou plusieurs photo-détecteurs montés en vis-à-vis sur la partie fixe de façon à pouvoir détecter la position de la LED, et donc, la position de la partie amovible 204A vis-à-vis de la partie fixe 204B.

Plus généralement, les moyens de commande locaux 204 du dispositif de contrôle 200 sont aptes à recevoir des commandes de réglage d'un ou de plusieurs des paramètres de contrôle de la pièce à main 218, ces commandes étant émises par l'opérateur en actionnant les moyens de commande locaux 204 (en tournant le bouton magnétique à effet hall par exemple).

Dans cet exemple, le dispositif de contrôle 200 est également équipé d'une pédale de commande 222 permettant à l'opérateur de commander la mise en marche et l'arrêt de la pièce à main 218.

Par ailleurs, la carte mère 202 comprend un module de sélection 216 apte à sélectionner soit les moyens de commande locaux 204, soit les moyens de communication sans fil 213 pour le réglage du ou des paramètres de contrôle. Le module de sélection 216 constitue ici des moyens de sélection au sens de l'invention.

Le module de contrôle 214 est configuré pour régler les paramètres de contrôle de la pièce à main chirurgicale 218 en fonction de la sélection faite par le module de sélection 216. Autrement dit, le module de contrôle 214 ne prend en compte que les commandes de réglage provenant des moyens sélectionnés par le module de sélection 216, à savoir : les moyens de communication sans fil 213 ou, alternativement, les moyens de commande locaux 204.

A tout moment, seul l'un parmi les moyens de communication sans fil 213 et les moyens de commande locaux 204 peut être sélectionné par les moyens de sélection 216 (i.e. la sélection simultanée des moyens 213 et 204 n'est pas possible).

Dans ce premier mode de réalisation, les moyens de commande locaux 204 comprennent des moyens de détection 206 de la présence de la partie amovible 204A sur le dispositif de contrôle 200. Autrement dit, les moyens de détection 206 sont aptes à détecter la présence (et donc *a contrario* l'absence) de la partie amovible 204A sur la partie fixe 204B.

Les moyens de détection 206 peuvent par exemple comporter des moyens optiques (un photodétecteur par exemple) permettant de détecter quand la partie amovible 204A est montée en position de travail sur la partie fixe 204B des moyens de commandes locaux 204.

Toujours dans ce premier mode de réalisation, les moyens de sélection 216 sont configurés pour sélectionner automatiquement les moyens de commande locaux 204 pour le réglage des paramètres de contrôle lorsque la présence de la partie amovible 204A sur la partie fixe 204B est détectée par les moyens de détection 206. Dans ce cas, le module de contrôle 214 prend alors en compte les commandes de réglage émises depuis les moyens de commandes locaux 204 pour régler les paramètres de contrôle de la pièce à main 218.

En revanche, lorsque les moyens de détection 206 détectent l'absence de la partie amovible 204A (lors du nettoyage du dispositif de contrôle 200 par exemple), les moyens de sélection sélectionnent automatiquement les moyens de communication sans fil 213 pour le réglage des paramètres de contrôle de la pièce à main 218. Dans ce cas, le module de contrôle 214 prend uniquement en compte les commandes de réglage reçues par les moyens de communication sans fil 213 en provenance du terminal distant 208 via la liaison de communication sans fil 220.

Comme indiqué précédemment, lorsque la partie amovible 204A des moyens de commande locaux 204 sont présents sur le dispositif de contrôle 200, seules les commandes issues du bouton 204 sont prises en compte pour le réglage des paramètres. On notera que lorsque les moyens de commandes locaux 204 sont sélectionnés par les moyens de sélection 216, la liaison de communication sans fil (de type bluetooth™ dans cet exemple) n'est pas nécessairement coupée.

Selon une variante préférée, la liaison de communication sans fil 220 est maintenue même lorsque la présence de la partie amovible 204A est détectée par les moyens de détection 206. Cette variante permet avantageusement d'éviter des temps d'attente indésirable résultant du délai de rétablissement (plus ou moins long) de la liaison de communication 220 lorsque la partie amovible 204A n'est plus présente sur le dispositif de contrôle 200. Selon cette variante préférée, lorsque les moyens de commande locaux sont sélectionnés (car la partie amovible 204A est présente sur le dispositif de contrôle 200), les commandes reçues par les moyens de communication sans fil 213 sont simplement ignorées par la carte mère 202 (ou éventuellement par la carte fille 212).

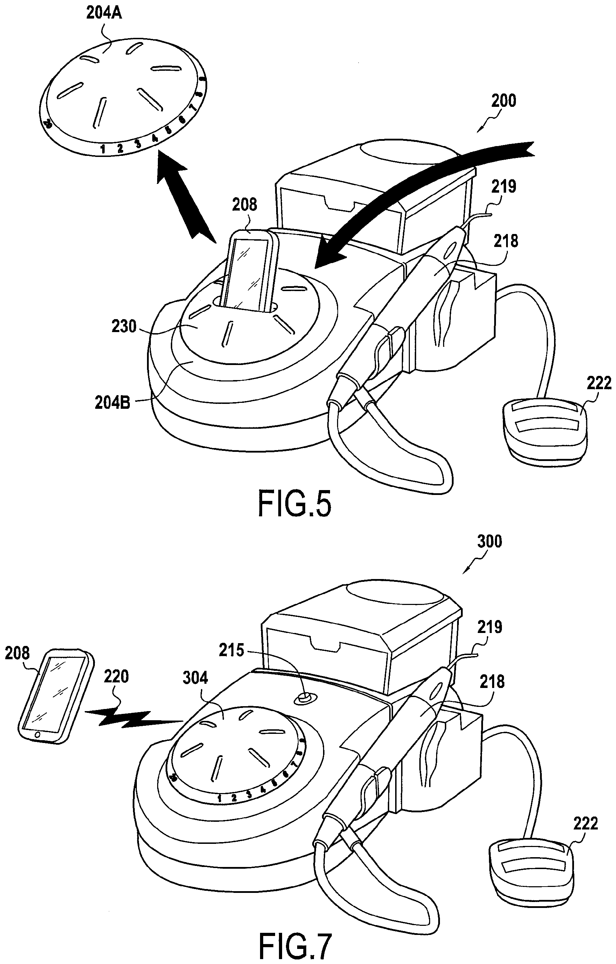

Selon une variante représentée en **figure 5****,** le terminal distant 208 peut être disposé sur un socle 230 qui est lui-même positionnable en lieu et place de la partie amovible 204A.

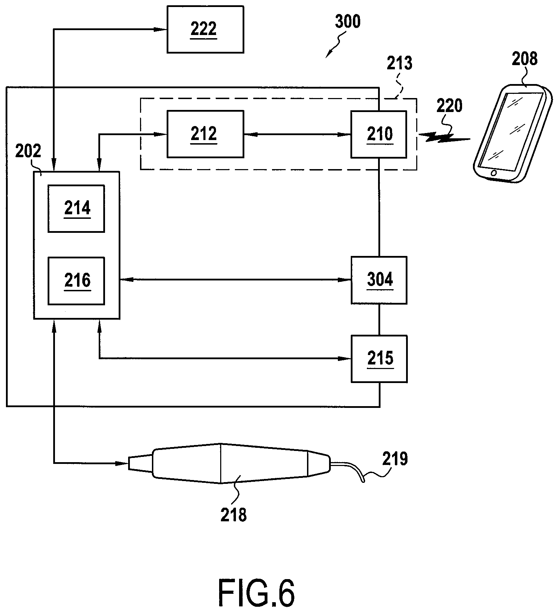

Un dispositif de contrôle 300 selon un deuxième mode de réalisation de l'invention est à présent décrit en référence aux **figures 6** **et** **7****.**

Ce dispositif de contrôle 300 comporte en majorité les mêmes éléments que ceux décrits ci-dessus en référence au dispositif de contrôle 200. Les éléments communs entre les dispositifs 200 et 300 portent donc les mêmes références et ne sont pas à nouveau décrits.

Le dispositif de contrôle 300 diffère du dispositif 200 précédemment décrit en ce que les moyens de commande locaux 304 ne comprennent pas de partie amovible. Dans ce deuxième mode de réalisation, les moyens de commande locaux prennent la forme d'un bouton 304 dont la rotation par rapport au boîtier du dispositif de contrôle 300 permet le réglage d'un ou de plusieurs paramètres de contrôle de la pièce à main 218. D'autres formes de commande locale sont toutefois envisageables.

Dans ce deuxième mode de réalisation, les moyens de commande locaux 304 sont donc dépourvus des moyens de détection 206 de présence comme précédemment décrit dans le premier mode de réalisation.

Le dispositif de contrôle 300 comportent en outre un commutateur 215 prenant ici la forme d'un bouton poussoir disposé sur le boîtier (d'autres formes de commutateur pourront toutefois être envisagés par l'homme du métier). Ce commutateur 215 est activable manuellement par l'opérateur et permet, par son actionnement, de sélectionner soit les moyens de commande locaux 304, soit les moyens de communication sans fil 213 pour le réglage de chaque paramètre de contrôle.

L'invention permet avantageusement de sécuriser le réglage des paramètres de contrôle de la pièce à main chirurgicale 218. Grâce à l'invention, il est en particulier toujours possible pour l'opérateur de reprendre rapidement le contrôle de la pièce à main 218 lorsqu'une défaillance survient au niveau de la communication sans fil 220 ou lors d'une quelconque situation d'urgence (comme expliqué précédemment, les situations d'urgence peuvent être d'origines multiples).

Le dispositif de contrôle de l'invention permet avantageusement de définir des niveaux de priorité entre les moyens de commande locaux 204 et les moyens de commande distants 208 : en cas de défaillance par exemple dans la réception ou le traitement des commandes de réglage émises par les moyens de commande distants 208, il est toujours possible pour l'opérateur de reprendre la main sur le contrôle de la pièce à main 218.

Dans le cas du premier mode de réalisation (dispositif de contrôle 200), dès que la partie amovible 204A est en position de travail, les moyens de commande locaux 204 ont la priorité sur les moyens de commande distants 208, i.e. les moyens de commandes locaux sont sélectionnés par les moyens de sélection 216. En conséquence, les commandes émises par le terminal 208 sont ignorées, seules les commandes provenant du bouton 204 étant alors prises en compte pour le réglage de la pièce à main 218.

Dans le cas du deuxième mode de réalisation (dispositif de contrôle 300), l'opérateur peut à tout moment commander, à l'aide du commutateur 215, la sélection soit des moyens de commande locaux 304, soit des moyens de communication à distance 213. Ainsi, en cas de défaillance de la liaison de communication sans fil 220 ou de tout autre situation d'urgence, l'opérateur peut rapidement reprendre la main sur la pièce à main chirurgicale 218 en sélectionnant le bouton 304 pour le réglage des paramètres de contrôle. De cette manière, le dispositif de contrôle 200 est forcé d'ignorer toutes commandes pouvant provenir des moyens de commande distants via la liaison de communication sans fil 220.

## Revendications

1. Dispositif de contrôle (200 ; 300) d'une pièce à main chirurgicale (218), apte à contrôler la pièce à main chirurgicale selon au moins un paramètre de contrôle, le dispositif de contrôle comprenant :
- des moyens de commande locaux (204 ; 304) présents sur ledit dispositif de contrôle ;
- des moyens de communication sans fil (213) aptes à communiquer avec des moyens de commande distants par une liaison de communication sans fil (220), les moyens de commande locaux et les moyens de communication sans fil étant aptes à recevoir des commandes de réglage de chaque paramètre de contrôle ; et
- des moyens de sélection (216) pour sélectionner soit les moyens de commande locaux (204 ; 304) soit les moyens de communication sans fil (213) pour le réglage de chaque paramètre de contrôle.

2. Dispositif de contrôle selon la revendication 1 dans lequel les moyens de commande locaux (204) comprennent une partie amovible (204A),
le dispositif de contrôle comprenant en outre des moyens de détection (206) de la présence de ladite partie amovible (204A) sur le dispositif de contrôle (200),
les moyens de sélection (216) étant configurés pour sélectionner automatiquement les moyens de commande locaux (204) pour le réglage de chaque paramètre de contrôle en cas de détection de la présence de ladite partie amovible (204A).

3. Dispositif de contrôle selon la revendication 1, dans lequel les moyens de sélection (216) comprennent un commutateur (215) activable manuellement pour sélectionner soit les moyens de commande locaux (304) soit les moyens de communication sans fil (213) pour le réglage de chaque paramètre de contrôle.

4. Dispositif de contrôle selon l'une quelconque des revendications 1 à 3, dans lequel la liaison de communication sans fil (220) est de type bluetooth™.

5. Dispositif de contrôle selon l'une quelconque des revendications 1 à 4, dans lequel les moyens de commande locaux (204) comprennent un bouton magnétique à effet hall.

6. Dispositif de contrôle selon l'une quelconque des revendications 1 à 5, dans lequel la pièce à main comprend un transducteur piézoélectrique, ledit au moins un paramètre comprenant au moins l'un parmi une puissance électrique fournie à la pièce à main, un débit d'irrigation de la pièce à main et l'activation ou désactivation de la pièce à main.

7. Appareil de contrôle d'une pièce à main chirurgicale comprenant :
- un dispositif de contrôle (200 ; 300) selon l'une quelconque des revendications 1 à 6 ;
- une pièce à main (218) dont les paramètres de contrôle sont réglés par ledit dispositif de contrôle ; et
- des moyens de commande distants (208) aptes à communiquer avec les moyens de communication sans fil (213) du dispositif de contrôle par une liaison sans fil (220).

8. Appareil de contrôle selon la revendication 7, dans lequel les moyens de commande distants (208) sont un module de communication de type téléphone mobile ou tablette numérique.

## Patentansprüche

1. Vorrichtung zur Steuerung (200; 300) eines chirurgischen Handstücks (218), die geeignet ist, das chirurgische Handstück entsprechend wenigstens einem Steuerparameter zu steuern, wobei die Steuervorrichtung umfasst:
- lokale Steuermittel (204; 304), die an der Steuervorrichtung vorhanden sind,
- Mittel zur drahtlosen Kommunikation (213), die geeignet sind, mit entfernten Steuermitteln über eine drahtlose Kommunikationsverbindung (220) zu kommunizieren, wobei die lokalen Steuermittel und die Mittel zur drahtlosen Kommunikation geeignet sind, Befehle zur Einstellung eines jeden Steuerparameters zu empfangen, und
- Auswahlmittel (216), um entweder die lokalen Steuermittel (204; 304) oder die Mittel zur drahtlosen Kommunikation (213) für die Einstellung eines jeden Steuerparameters auszuwählen.

2. Steuervorrichtung nach Anspruch 1, bei der die lokalen Steuermittel (204) einen lösbaren Teil (204A) umfassen,
wobei die Steuervorrichtung ferner Mittel zur Erfassung (206) des Vorhandenseins des lösbaren Teils (204A) an der Steuervorrichtung (200) umfasst,
wobei die Auswahlmittel (216) dazu ausgelegt sind, die lokalen Steuermittel (204) für die Einstellung eines jeden Steuerparameters im Fall der Erfassung des Vorhandenseins des lösbaren Teils (204A) automatisch auszuwählen.

3. Steuervorrichtung nach Anspruch 1, bei der die Auswahlmittel (216) einen manuell aktivierbaren Schalter (215) umfassen, um entweder die lokalen Steuermittel (304) oder die Mittel zur drahtlosen Kommunikation (213) für die Einstellung eines jeden Steuerparameters auszuwählen.

4. Steuervorrichtung nach einem der Ansprüche 1 bis 3, bei der die drahtlose Kommunikationsverbindung (220) vom Typ Bluetooth™ ist.

5. Steuervorrichtung nach einem der Ansprüche 1 bis 4, bei der die lokalen Steuermittel (204) einen Hall-Effekt-Magnetknopf umfassen.

6. Steuervorrichtung nach einem der Ansprüche 1 bis 5, bei der das Handstück einen piezoelektrischen Wandler umfasst, wobei der wenigstens eine Parameter wenigstens einen aus einer dem Handstück gelieferten elektrischen Leistung, einem Spüldurchsatz des Handstücks und der Aktivierung oder Deaktivierung des Handstücks umfasst.

7. Gerät zur Steuerung eines chirurgischen Handstücks, umfassend:
- eine Steuervorrichtung (200; 300) nach einem der Ansprüche 1 bis 6,
- ein Handstück (218), dessen Steuerparameter durch die Steuervorrichtung eingestellt werden, und
- entfernte Steuermittel (208), die geeignet sind, mit den Mitteln zur drahtlosen Kommunikation (213) der Steuervorrichtung über eine drahtlose Verbindung (220) zu kommunizieren.

8. Steuergerät nach Anspruch 7, bei dem die entfernten Steuermittel (208) ein Kommunikationsmodul vom Typ Mobiltelefon oder digitalem Tablet sind.

## Claims

1. A control device (200; 300) for controlling a surgical handpiece (218), the device being suitable for controlling the surgical handpiece according to at least one control parameter, the control device comprising:
• local control means (204; 304) present on said control device;
• wireless communication means (213) suitable for communicating with remote control means by a wireless communication link (220), the local control means and the wireless communication means being suitable for receiving instructions to adjust each control parameter; and
• selector means (216) for selecting either the local control means (204; 304) or the wireless communication means (213) for adjusting each control parameter.

2. A control device according to claim 1, wherein the local control means (204) include a removable portion (204A);
• the control device also comprising detector means (206) for detecting the presence of said removable portion (204A) on the control device (200);
• the selector means (216) being configured to select the local control means (204) automatically for adjusting each control parameter in the event of the presence of said removable portion (204A) being detected.

3. A control device according to claim 1, wherein the selector means (216) comprise a manually-operable switch (215) for selecting either the local control means (304) or the wireless communication means (213) for adjusting each control parameter.

4. A control device according to any one of claims 1 to 3, wherein the wireless communication link (220) is of TM the Bluetooth type.

5. A control device according to any one of claims 1 to 4, wherein the local control means (204) comprise a Hall effect magnetic button.

6. A control device according to any one of claims 1 to 5, wherein the handpiece includes a piezoelectric transducer, said at least one parameter comprising at least one of the following: an electrical power level to be delivered to the handpiece; an irrigation flow rate for the handpiece; and activating or deactivating the handpiece.

7. An apparatus for controlling a surgical handpiece, the appliance comprising:
• a control device (200; 300) according to any one of claims 1 to 6;
• a handpiece (218) having control parameters that are adjusted by said control device; and
• remote control means (208) suitable for communicating with the wireless communication means (213) of the control device via a wireless link (220).

8. A control appliance according to claim 7, wherein the remote control means (208) are a communications module of the mobile telephone or digital tablet type.
